# EUROPEAN PATENT SPECIFICATION

(11) **EP 3 336 817 B1**
(45) Date of publication and mention of the grant of the patent: **09.10.2019**
(21) Application number: 16462011.4
(22) Date of filing: 22.12.2016
(51) Int. Cl.: G08B 21/24

(54) **METHOD, APPARATUS AND COMPUTER PROGRAM PRODUCT FOR HAND DISINFECTION QUALITY CONTROL**
VERFAHREN, VORRICHTUNG UND COMPUTERPROGRAMMPRODUKT ZUR QUALITÄTSKONTROLLE DER HANDDESINFEKTION
PROCÉDÉ, APPAREIL ET PRODUIT DE PROGRAMME INFORMATIQUE POUR LE CONTRÔLE DE LA QUALITÉ DE LA DÉSINFECTION DES MAINS

(30) Priority: 14.12.2016 HU 1600669
(43) Date of publication of application: 20.06.2018
(73) Proprietor: HandinScan Zrt., 4025 Debrecen (HU)
(72) Inventor: Haidegger, Tamás Péter, H-1122 Budapest (HU); Szerémy, Péter, H-7630 Pécs (HU); Lehotsky, Ákos, H-1107 Budapest (HU); Major, Gergely, H-2890 Tata (HU); Takács, Bence, H-8743 Zalaszabar (HU); Róna, Péter, H-2051 Biatorbágy (HU)
(74) Representative: Harangozo, Gabor

(56) References cited:
- US-A1- 2012 173 274
- US-A1- 2013 215 245
- US-A1- 2014 333 744

## Description

### TECHNICAL FIELD

The invention relates to a method, an apparatus and a computer program product for hand disinfection quality control.

### BACKGROUND OF THE INVENTION

Hospital-acquired infections (HAI), also referred to as healthcare-associate infections or nosocomial infections, are one of the leading causes of death in the United States and Europe. The major source of HAI is the improper hand disinfection. HAI generate unnecessary expenses, reduces the quality of life of the patients, prolongs recovery and promotes the resistance of pathogens against antibiotics. Furthermore, hand hygiene has outstanding importance at nursing homes, veterinarian clinics, clean manufacturing plants, biotechnological production, food service stations and in the hospitality industry.

The applied methods of hand disinfection have been widely discussed in recent studies such as in Behre M. et al., "Measurement and feedback of infection control process measures in the intensive care unit: impact on compliance" (American Journal on Infection Control, Vol. 34, no. 8, 2006, pp. 537-539), World Health Organization (WHO) Guidelines on Hand Hygiene in Health Care (WHO First Global Patient Safety Challenge Clean Care is Safer Care, 2011) and (Centers for Disease Control and Prevention) CDC guidelines (How-to Guide: Improving Hand Hygiene A Guide for Improving Practices among Health Care Workers, CDC, 2006). However, despite the numerous disinfection stations and the spread of antibacterial soaps, the insufficient hand washing remains a major problem in health care and causes several infection-related problems at general households as well. In the medical environment, appropriate hand disinfection is required to keep HAI rates low, especially as the new mutant germs - such as the Methicillin-resistant Staphylococcus aureus (MRSA), New Delhi metallo-beta-lactamase 1 (NDM-1), Carbapenem-resistant enterobacteriaceae (CPE) - show high resistance to antibiotic treatment. Also in the general practice, proper hand hygiene helps to maintain a healthy life, and to prevent or reduce the spread of epidemics (e.g., Severe Acute Respiratory Syndrome (SARS), Influenza A (H1N1), etc).

Document US 2013/0215245 A1 discloses a method for hand disinfection quality control, the method comprising the steps of providing a hand disinfectant medium containing light reflecting particles responsive to light mostly outside the visible spectral range of light, applying the hand disinfectant medium on the hands in a prescribed manner for a prescribed time, and illuminating the treated hands by means of a light source providing light in a spectral range for activating the reflecting particles. The method further comprises the steps of recording digital images of the hands from both sides, and evaluating the recorded images by a computer program, in order to determine the extent of cleanness of the hands. The aforementioned document also discloses an apparatus for implementing the aforementioned method and assessing hand disinfection quality. The apparatus comprises a rigid case with side walls enclosing a light source for providing light of a predetermined spectral range mostly outside the visible range. The case is further provided with an opening on the front wall for receiving the hands to be exposed to the light of the light source. An imaging device is also attached to the case for taking images of the illuminated hands.

The above described method and apparatus have the drawback that the use of light reflective particles in the disinfectant medium allows only a negative signaling method, wherein the presence of the disinfectant medium on the hands provides high intensity in the recorded images, whereas the untreated areas appears with low intensity in the images and therefore these regions are often hardly distinguishable from the background of the recorded image. This means that the untreated regions are just hard to exactly identify.

Another drawback is that the use of UV fluorescent molecules requires the use of UV radiation and thus assumes the application of expensive UV sources.

A further drawback of the above method is that the light reflective particles, which are typically UV-reflective particles, can also be easily observed visually, thus the light reflective particles that are not removed from the hands after their treatment, can still be seen under UV light. This might be inconvenient for those who must stay at a place where UV light is necessary to be used (e.g., in clubs, tanning salons, in front of some windows, etc), moreover it prevents further objective measurements until the UV-reflective particles are completely removed from the hand.

Document US 2014/0333744 A1 discloses a system and a method for monitoring potential spread of an infectious agent in an environment. The method includes providing a detectable marker/tracer for emplacement on one or more body regions of a subject at a time when the subject is entering the environment; identifying a unique tag/identifier associating the detectable marker/tracer with the subject; observing with a camera the marker/tracer in the environment; and detecting a transfer of the marker/tracer from the one or more body regions of the subject to one or more surfaces of the environment.

Document US 2012/0173274 A1 discloses a validated healthcare facility cleaning and sanitizing system providing a comprehensive and systematic approach to cleaning and sanitizing practices at a hospital or other healthcare facility. The validated hospital cleaning system identifies hospital vectors of contamination; that is, sources through which hospital acquired infections (HAIs) may be spread. The validated system defines a plurality of modules within a healthcare facility, each having an associated cleaning process map designed to meet the particular cleaning and/or sanitizing needs and challenges faced by that module. Various stages of the cleaning process map include validation points, at which certain parameters designed to ensure proper cleaning and/or sanitizing of the module are verified. Neither of the above two documents involves hand disinfection quality control on the basis of processing images taken of hands previously treated with a specific disinfectant material.

It is an object of the present invention to provide an improved technology for hand disinfection qualtity control, wherein the untreated regions of the hand can be more accurately identified in the recorded images of the hands, while employing a novel imaging technique and offering new solutions for system integration.

It is a further object of the present invention to provide an improved technology for hand disinfection qualtity control, which uses special materials for the disinfectant medium that remain substantially invisible for humans after the treatment of the hands under either normal conditions or even UV light.

### SUMMARY OF THE INVENTION

These and other objects are achieved by providing an apparatus for hand disinfection quality control, the apparatus comprising a housing, wherein within the housing the apparatus comprises: a hand inspection cavity formed on a front face of the housing and adapted for receiving a hand of a user; a plurality of light sources arranged to direct ultraviolet, UV, or visible light or infrared, IR, radiation to an internal space of said hand inspection cavity; at least one digital camera arranged in the hand inspection cavity and configured to capture images in the UV, visible or IR spectrum; a hand proximity sensor configured to detect presence of a hand of a user within said hand inspection cavity; a container for storing a disinfectant medium; a dispenser for a metered dosage of the disinfectant medium; a processor unit; and an internal data storage unit; wherein on the outer surface of the housing the apparatus further comprises input means for locally controlling the operation of the apparatus; and display means; wherein the disinfectant medium contains an UV or IR absorbing material, or a phosphorescent material or a chemiluminescent material; and wherein the processor unit is configured
- to generate intensity information for the hand from the images recorded by the at least one camera, said intensity providing information on the extent of the hand surface area covered by the disinfectant medium,
- to process said intensity information using a predetermined or user-specific evaluation algorithm for determining the acceptable level of hand sanitation, and
- based on said intensity information processing, to determine whether or not the quality of disinfection of the inspected hand is acceptable.

The above objects are further achieved by providing a method of performing hand disinfection quality control on the above apparatus, the method comprising the steps of: detecting the presence of a user's hand and the posture of the hand; dispensing a hand disinfectant medium comprising UV, IR, or visible light absorbing material onto the hand of the user in a specific dose; illuminating the hand by the UV, visible or IR light; recording one or more digital images of the hand by from at least two sides of the hands; based on the recorded images, generatting intensity information for the hand, wherein said intensity information provides information on the extent of hand surface area covered by the disinfectant medium; comparing the generated intensity information with threshold intensity information which corresponds to an acceptable hand sanitation level; and based on a result of the comparison, determining whether or not the quality of the hand disinfection is acceptable.

Finally, the above objects are also achieved by providing a computer program product containing instructions which, when executed by the apparatus according to the invention, cause the apparatus to perform the method according to the invention.

The method and the apparatus according to the present invetion allow an accurate and objective identification of the disinfected areas on a treated hand, and therefore the determiniation of the overall quality of hand sanitizing in accordance with the respective sanitization requirements.

### BRIEF DESCRIPTION OF THE DRAWINGS

Further objects, modifications and improvements of the present invention will be clear from the description of the preferred embodiments of the method and the apparatus according to the invention in conjunction with the accompanying drawings, in which:
FIG. 1 illustrates a preferred embodiment of the apparatus according to the invention in a schematic perspective view,
FIG. 2 is a schematic block diagram showing the major functional elements of the apparatus according to the invention, wherein some optional elements are also indicated by dotted lines,
FIGs. 3A and 3B show images of a partially treated hand recorded in the spectrum of the visible light and in the UV spectrum, respectively, and
FIGs. 4A and 4B are flow diagrams of the steps of the method in accordance with the present invention.

The core idea of the invented technology is the use of a special lightabsorbing material in the disinfectant medium, instead of using light-reflecting materials. The light-absorbing material may be any suatable material, which absorbs the light in a range mostly outside the visible spectral range of light. Preferably, the light-absorbing materials suitable for the present invention include ultraviolet (UV) and infrared (IR) light-absorbing materials.

Absorption-based imaging is an important distinguishing feature from any known technology, since it allows for the detailed analysis of the region-of-interest on the hands, as there is no additional layer of photoactive or photoreflective materials covering the skin. Absorption in the skin is also happening faster, thus the visibility of the treated areas decreases quickly. This is advantageous, since the hand hygiene performane measurement itself should not interfere with the hands for long, supporting additional objective assessments later on.

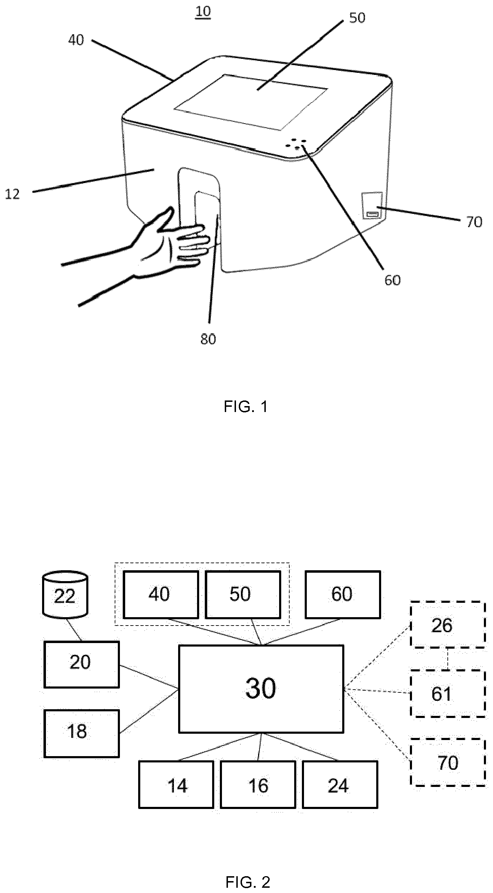

FIGS 1 and 2 depict a hand disinfection quality control apparatus 10 according to the invention, which is preferably carried out in the form of a compact and mobile tool for the objective assessment of hand disinfection quality. The apparatus 21 comprises a housing 12 that is preferably designed as a rigid, wall-mountable box. The housing 12 of the apparatus may be made of a light metal, such as aluminum, or any plastic material with similar properties to ensure rigidity while facilitating mobility of the apparatus due to its light overall weight, and is easy to clean.

The core idea of the invented technology is the use of phosphorescent molecules or particles or pigments or a chemiluminescent material in the disinfectant medium, instead of using UV-reflecting fluorescent materials. The phosphorescent or chemiluminescent material may be any suitable material, which absorbs the light in the UV, IR or visible spectral range of light.

Within said housing 12, the apparatus 10 comprises built-in light sources 14, one or more digital cameras 16, a hand proximity sensor 18, a dispenser 20 including a pump to dispense the disinfectant medium, a container 22 for storing the disinfectant medium, a data storage unit 24 and a processor unit 30 configured to operate the whole apparatus. Preferably, the apparatus 10 further comprises an interface unit 70 for connecting the apparatus with another external device, like a personal computer or a laptop, directly or through a communication nerwork, for locally or remotely supervising operation of the apparatus and for allowing external evaluation of the utiliziation of the apparatus by the users. In case the apparatus comprises a wired interface unit 70, an interface connector, e.g., a USB port, is provided on a wall of the apparatus. Preferably, the apparatus may contain a user proximity sensor 61 to trigger a measurement or initiate an action on its screen.

In a particularly preferred embodiment of the apparatus 10, an RFID-based or other touchless personal identification module 26 may be provided. In this embodiment, the personal identification modul 26 is activated by the user proximity sensor 61 when a user approaches the apparatus to a certain distance.

The surfaces of the walls of the apparatus may be treated with an anti-bacterial coating or may be made of an anti-bacterial material for minimizing infection transfer from one user to another user in case of intensive use of the apparatus by a plurality of users.

On its outer surface, the apparatus further comprises an input means 40 for locally controlling the operation of the apparatus by a user, a display means 50 for displaying operative information for the user, one or more status indicators 60 for producing visual or audible status signals representing operating modes of the apparatus and/or disinfection quality control test results.

As shown in FIG 1, on the front side of the apparatus 10, an inspection cavity 80 (or slot) is formed to receive one hand of a user.

Preferably, the light sources 14 are UV or IR light sources, radiating light substantailly in the invisible range of the spectrum. UV LEDs may be used to reduce energy consumption. Any other type of UV or IR light sources may be used for the light sources 14 as well that have no health risk at their operational power. The arrangement of the light sources 14 are preferably optimized to minimize the presence of shadows for the digital camera or cameras 16, each of which is arranged within said cavity 80.

For phosphorescent or chemiluminescent materials, the light sources 14 operate in a spectrum in which the particles of said materials absorb the illuminating light.

The cameras 16 used in the apparatus 10 may comprise UV or IR light sensitive elements and thus capable of recording images in the ultraviolet or infrared spectrum, resepctively. Concerning the infrared spectrum, a wide range of infrared spectrum, also including the near-infrared (NIR) range, may be suitable for use in the apparatus according to the present invention.

The cameras 16 may be highly sensitive in the visible range or in the the near-infrared (NIR) range.

The cameras 16 are configured to focus on the whole hand, and preferably, the digital images are taken with a fixed aperture and a fixed exposure time that are controlled by the processor unit 30 of the apparatus.

A preferred embodiment of the apparatus may employ reflected ultraviolet photography for recording images of the hand, by using light from the ultraviolet (UV) spectrum only. In this embodiment the hand being subject to inspection is illuminated by UV light sources 14. An UV light transmitting optical element (e.g. glass, plastic or gelatin filter(s), absorptive filter; interference filter or dichroic filter) is placed on the lens of the digital camera(s) 16, either in front of or behind the lens, said optical element allowing the ultraviolet light to pass, while absorbing or blocking or reflecting all visible and infrared light. For UV photography, it is necessary to use specially developed lenses having elements made from special glass, fused silica quartz, fluorite or special plastic without UV coating. Most types of glass will allow long wave UV to pass, but absorb all the other UV wavelengths, usually in the UV range up to about 350 nm. A special sensor or a plurality of sensors may be required to detect the reflected UV radiation in each of the digital cameras 16.

The UV transmitting filter may contain one or more optical elements, selective UV transmitting or nonselective UV transmitting optical element in combination with elements capable of eliminating the visible and/or infrared spectrum. These optical elements may be used, for example, to limit the passing wavelengths to the band of the whole UV range (UVC, UVB and UVA) but not longer than 400 nm, to limit the passing wavelengths to a band within the UV range.

The UV-capable lens may be configured to be able to pass the whole UV spectrum or only certain parts thereof.

The UV-capable sensors of the camera(s) 16 are preferably configured to record in the whole UV spectrum or in any part thereof.

When a phosphorescent material is used in the disinfactant medium, a preferred embodiment of the apparatus may use visible light for illumination of the hand and NIR photography for recording images of the hand. An IR light transmitting optical element (e.g. glass, plastic or gelatin filter(s), absorptive filter; interference filter or dichroic filter) may be placed on the lens of the digital camera(s) 16, either in front of or behind the lens, said optical element allowing the incident light to pass, while absorbing or blocking or reflecting all other wavelengths.

When a chemiluminescent material is used in the disinfactant medium, a preferred embodiment of the apparatus may comprise a light transmitting optical element (e.g. glass, plastic or gelatin filter(s), absorptive filter; interference filter or dichroic filter) placed on the lens of the digital camera(s) 16, either in front of or behind the lens, said optical element allowing the incident light to pass, while absorbing or blocking or reflecting all other wavelengths.

Images taken in visible light (e.g. under the illumination provided by white LEDs) may support the imaging process.

In order to assure an acceptable speed of image processing (preferably within a few seconds), the captured images may be downsized (without significantly affecting the segmentation accuracy), then filtering may be carried out. Out of the three intensity channels (corresponding to red, green and blue (RGB) or HSV channels), one may be used for finding the region of interest (segmenting the entire hand) and another one may be used for image processing, to classify the pixels (segmenting the clean areas). In order to separate the pixels belonging to treated and untreated regions of the hand, a quick segmentation algorithm is to be applied that may be fed with the histogram of a single-channel input image. Finally, pixel-based filtering, region-based filtering and weighting are used to produce the final image infromation which can be processed for sanitation quality control. This final image information may include, for example, a visual overlay of the treated areas and the entire hand in 2D or in 3D with associated numeric details, the percentage of the trated areas relative to the entire hand surface, and an objective quality score for the efficiency of the whole hand disinfection. These and other useful pieces of information of the disinfection quality control processing may be displayed on the display 50. Optionally, sound effects may also be generated to present audible indications of pass/fail events.

The walls of the inspection cavity 80 are preferably made of an UV or IR absorbing material or covered by an UV or IR absorbing material, or at least they have their UV or IR reflection properties significantly different from those of the surface of a human hand (i.e. skin). For example, a mat (i.e. poorly reflecting) black cover of the cavity walls helps to reduce the environmental disturbing effects during image acquisition.

Due to the above mentioned features of the inspection cavity 80 of the apparatus 10 according to the invention, the UV or IR light absorbing areas of the treated hand will be clearly distinguishable from the untreated, UV or IR reflecting areas of the hand in the images recorded by the cameras 16.

The hand proximity sensor 18 is adapted to detect the presence of a hand within the inspection cavity 80. The hand proximity sensor 18 may be any type of proximity sensor, including optical sensors, ultrasound sensors, radio frequency sensors an so on. The hand proximity sensor 18 is preferably integrated into a wall of the inspection cavity 80 or arranged adjacent to the cavity 80 as an element within the housing 12.

The apparatus 10 may also be equipped with a user proximity sensor 61 to detect if someone approaches the appartus or stands in front of the apparatus. The user proximity sensor 61 is used to trigger the operation of the apparatus, for example, to start a training presentation of how to use the apparatus or how to correctly santize the user's hand. It is noted that these functions may be provided in other ways as well, for example by means of an activation button (not shown) mounted on the external wall of the case of the apparatus.

The dispenser 20 is used to dispense the disinfectant medium stored in the container 22 in predetermined doses by means of a calibrated pump. The disinfectant medium may be in the form of a liquid, a gel or a soap. The dispensed amount of the disinfectant medium may be determined on the basisof the identity of the user, the hand size, the location of use of the device or any other preference of the operator. Dispensing mehanisms suitable for electronically controlled, metered dosage of an agent are well known in the art. Preferably, the disinfectant medium is a special mixture of an UV light absorbing material and a conventionel alcohol-based disinfectant. Alternatively, infrared light absorbing material can also be used with a conventionel alcohol-based disinfectant for the hand disinfection quality control carried out by means of the apparatus according to the present invention. Alternatively, a phosphorescent material or a chemiluminescent material can also be used mixed with a conventionel alcohol-based disinfectant for the hand disinfection quality control carried out by means of the apparatus according to the present invention, assuming that they can also absorbe the light in the spectrum employed for the inspection.

The processor unit 30 is configured to operate the whole apparatus 10 by receiving signals from the hand proximity sensor 18 and the input means 40, and by controlling the operation of the light sources 14, the at least one camera 16, the display 50, the status indicators 60 and the dispenser 20. The processor uint 30 is also adapted for controlling the recording, recalling and transmitting of date from/to the internal data storage unit 24, or from/to any external data storage unit.

The input means 40 include any means suitable to input control infromation or other user data into the apparatus 10. In a particularly preferred embodiment of the apparatus accoding to the invention, the input means 40 and the display 50 may be provided in an integrated form, for example as a touch-screen, or may be formed as a part of a hand-held electronic device, such as a smart phone or a PC.

The images recorded by the camera(s) 16 may be transmitted to an external processor device, such as a personal computer or a portable notebook attached to the optional wired (e.g. USB) or wireless (e.g. WiFi, Bluetooth, ZigBeeetc.) interface unit 70 of the apparatus, and the external processor device may perform an automatic evaluation procedure, providing images of enhanced quality that highlights the difference between the treated areas and the untreated areas of the hand, and further provides overall quantitative and/or qualitative information with respect to the ongoing hand santiation process. Locally computed results and data may also be transmitted to an external processor and/or storage unit.

Beyond the visual presentation of the results of the hand sanitation process, the data resulted from the image processing may be forwarded to (through the optional interface unit 70) and stored in a central database, therefore an external processing device may further process and evaluate the hand sanitation information provided by the apparatus. For example, statistics over the hand hygiene performance of a plurality of users may be established and made available for an infection control staff or the management of a health care institute.

Furthermore, it may be useful to build an initial database of typical user hands to calibrate the algorithm for the individual hand and skin properties (such as tone, birth marks, vessels, etc.). This ensures the robustness of the evaluation procedure, and may also determine the required dosage of disinfectant medium to be dispensed at every use. Basically, each user's hands should be recorded in a completely untreated state (i.e. without any hand-rubbing applied thereon). On this kind of initial image, special skin features can be identified, and their locations may be stored relatively to the segmented hand of the user.

The wireless communication may allow the apparatus to automatically switch to preset parameters during the image processing, when a registered user is identified at using the apparatus. Otherwise, the image recording and processing software of the apparatus runs using a generic set of parameters (i.e. default mode), without specifically edged for the actual user.

According to a preferred embodiment, the apparatus may further comprise a user identification module for recording identity of the user whose hand is subject to disinfection quality control, wherein the user identification module may be based on the use of any one of the personal identification technologies including magnetic card, smart card, RFID, ultrasound or infrared (IR) identification.

The display 50 may be configured to present user-specific information, special educational materials, video training, preferably according to the given user identified by the user identification module.

Furthermore, the apparatus may be equipped with loud speakers for presenting audio instructions to the user during the hand disinfection process.

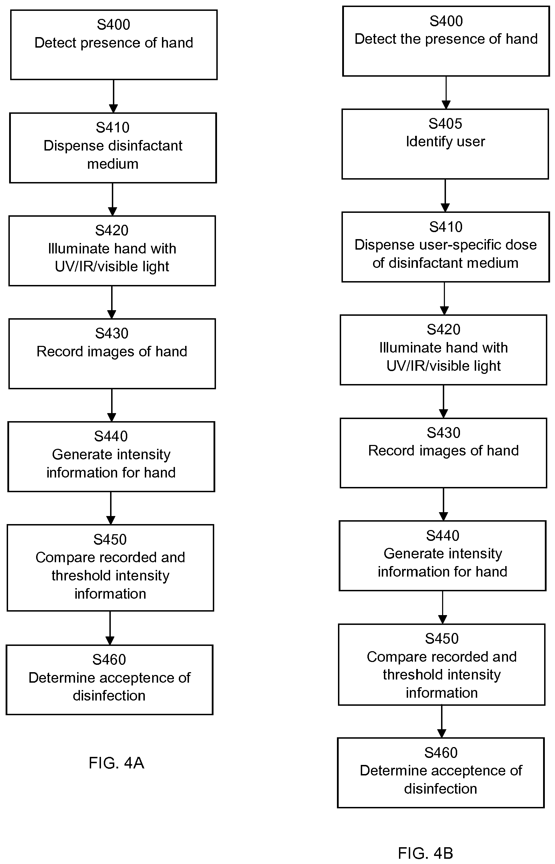

FIG 4A shows a flow diagram of the steps of the operation of the apparatus in accordance with the present invention. For the method, it is assumed that the user proximity sensor has already detected the presence of the user in front of the apparatus, or the user made a clear indication of its intention to use the device (e.g., by applying his or her RFID to the RFID reader or entering a personal identification (PIN) code on the touch screen).

In a first step S400, the presence of a user's hand within the inspection cavity is detected by means of the hand proximity sensor of the apparatus.

In a further step S410 of the method, a hand disinfectant medium comprising UV or IR light absorbing material is dispensed onto the hand of the identified user in a specific dosein response to the detection of the presence (or a required position) of the hand inserted into the inspection cavity. The amount of the dose of the medium can be read from the internal or external data storage unit, and forwarded to the pump of the dispenser, which dispenses the specified amount of disinfectant medium on the hand(s) of the user.

After the user has distributed the disinfectant medium on the entire surface of his or her hand and inserted it again into the inspection cavity of the apparatus, the inserted hand is illuminated by the UV, IR or other light sources in step S420 and one or more digital images of the hand are recorded by the UV or IR sensitive cameras in step S430, at least from two sides of the hands (e.g. palmar and dorsal). It is preferred that by using more than two cameras, for example four or six cameras, the entire surface of the hand can be appropriately scanned by the cameras, in order to allow a more reliable sanitation quality control. Alternatively, 3D stereo cameras or depth sensors may also be used to obtain a 3D image of the hands.

Based on the recorded images of the hand, intensity information is generated for the illuminated hand in Step S440 by means of the processing unit of the apparatus, wherein said intensity provides information on on the extent of hand surface area covered by the disinfectant medium. In a preferred embodiment of the method of the invention, the intensity information is a 2D or 3D intensity map of the illuminated hand showing the concentration distribution of the UV, visible light or IR absorbing particles over the scanned areas of the inspected hand. In this case the non-covered areas of the hand form the highest intentisity areas, whereas the areas covered by the disinfactant medium show lower intensity depending on the amount of the disinfectant medium applied on the specific surface regions. Alternatively, the intentity information may represent an overall intensity value of the illuminated hand.

Optionally, in step S405, the user may be identified by means of an RFID belonging to the user or other personal identification technology (e.g. iris scanning, inputting PIN code, etc.), as shown in Fig. 4B. In this embodiment of the method, i.e. when identification of the users is available, a user-specific dose of the disinfectant medium may be dispensed in step S410, as shown in Fig. 4B.

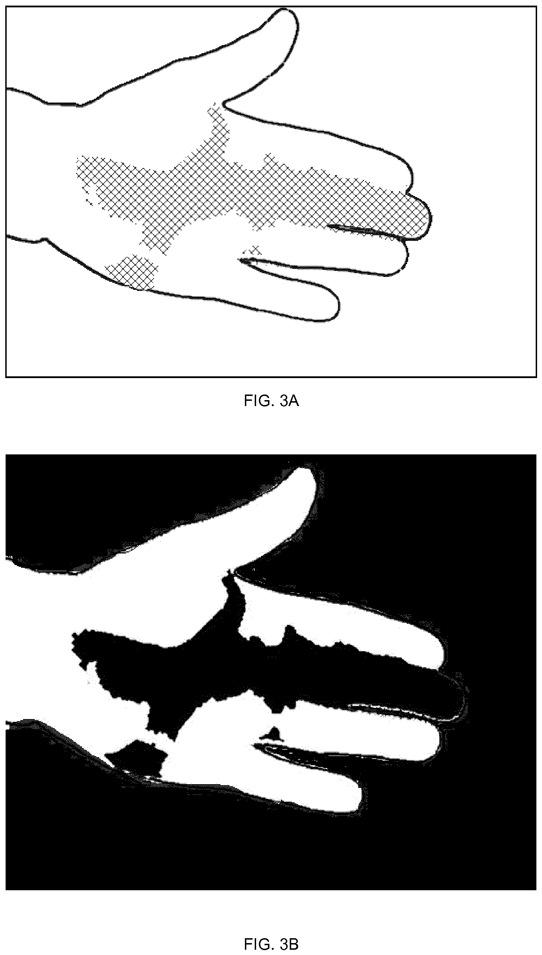

As an example, Figures 3A and 3B show images of a partially treated hand recorded in the spectrum of the visible light and in the UV spectrum, respectively. As shown in Fig. 3A, the treated (UV absorbing) areas of the hand can be hardly recognised in the visible light spectrum. However, as shown in Fig. 3B, the treated areas of the hand (covered by the disinfectant medium containing an UV absorbing material) and the background of the image (corresponding to the walls of the inspection cavity) have substantially no reflective intensity (appearing in dark), whereas the untreated areas of the hand have high reflective intensity (appearing in light), thereby definitely showing those parts of the hand where no sufficient amount of disinfectant medium has been applied on the hand.

Then in step S450, the generated intensity information is comapred by the processing unit with predetermined or user-specific threshold intensity information, which corresponds to an acceptable hand sanitation level of the current user. This step may include the comparison of a 2D or 3D intensity map of the illuminated hand with a corresponding threshold intensity map or the comparison of an overall intensity value of the illuminated hand with a corresponding threshold overall intensity value.

Finally, it is determined in step S460 whether the quality of disinfection is acceptable or not based on the comparison of the recorded intensity map and the threshold intensity map, or by other individualized software algorithms. The recorded image data may be further processed by the apparatus or the user may be infromed on the result of the disinfection quality control process, for example, by displaying the result of the hand disinfection quality control test for the user on the display of the apparatus, or generating a respective sound effect for the user about the success or failure of the santiation process. The recorded image data and/or the test results may be forwardedto an external processing device (e.g., a locally connected computer or a remote computer through a wired or wireless communiation network) using the interface unit of the apparatus.

The individual sanitation test results of the users may be used to build a database either locally in the apparatus or remotely, in an external database. The data stored in the data base may be used to a subsequent analysis of the users' sanitation activities and evaluate the sanitation test results on a statistical basis for making further decisions with respect to the overall hand disinfection quality control scheme of a hospital, a health center or any other institute.

The invention also relates to a computer program stored on a computer-readable medium, containing instructions that when executed by the apparatus according to the present invention cause the apparatus to carry out the above hand disinfection quality control method in accordance with the present invention.

## Claims

1. An apparatus (10) for hand disinfection quality control, the apparatus comprising
- a housing (12), wherein within the housing (12) the apparatus comprises:
- a hand inspection cavity (80) formed on a front face of the housing and adapted for receiving a hand of a user,
- a plurality of light sources (14) arranged to direct ultraviolet, UV, or visible light or infrared, IR, radiation to an internal space of said hand inspection cavity,
- at least one digital camera (16) arranged in the hand inspection cavity and configured to capture images in the UV, visible or IR spectrum,
- a hand proximity sensor (18) configured to detect presence of a hand of a user within said hand inspection cavity,
- a container (22) for storing a disinfectant medium,
- a dispenser (20) for a metered dosage of the disinfectant medium,
- a processor unit (30), and
- an internal data storage unit (24),
wherein on the outer surface of the housing (12) the apparatus further comprises:
- input means (40) for locally controlling the operation of the apparatus, and
- display means (50),
wherein the disinfectant medium contains an UV or IR absorbing material, or a phosphorescent material or a chemiluminescent material, and
wherein the processor unit (30) is configured
- to generate intensity information for the hand from the images recorded by the at least one camera (16), said intensity providing information on the extent of the hand surface area covered by the disinfectant medium,
- to process said intensity information using a predetermined or user-specific evaluation algorithm for determining the acceptable level of hand sanitation, and
- based on said intensity information processing, to determine whether or not the quality of disinfection of the inspected hand is acceptable.

2. The apparatus according to claims 1, wherein the walls of the inspection cavity (80) are made of an UV, visible light or IR absorbing material or covered by an UV, visible light or IR absorbing material, or they have their UV, visible light or IR reflection properties significantly different from those of the skin of a human hand.

3. The apparatus according to claim 1 or 2, wherein said input means (40) and said display means (50) are formed as an integrated touch-screen.

4. The apparatus according to any one of claims 1 to 3, further comprising one or more status indicator to produce visual or audible status signals representing operating modes of the apparatus and/or disinfection quality control test results.

5. The apparatus according to any one of claims 1 to 4, further comprising an interface unit (70) for connecting the apparatus to an external processor device through a wired or wireless interconnection.

6. The apparatus according to any one of claims 1 to 5, further comprising a user identification module to identify the user whose hand is subject to disinfection quality control.

7. The apparatus according to claim 6, wherein the user identification module is based on the personal identification technology selected from the group of: magnetic card, smart card, RFID, NFC, fingerprint, ultrasound or infrared (IR) identification.

8. The apparatus according to any one of claims 1 to 7, wherein a visual and audible alert means is connected to the apparatus, said alert means being configured to notify surrounding people if a user's hand hygiene performance has failed to meet the requirements.

9. The apparatus according to any one of claims 3 to 8, wherein it further contains a touch screen to provide access to its internal software for service and maintenance.

10. A method of performing hand disinfection quality control on an apparatus according to any of claims 1 to 9, the method comprising the steps of:
- detecting (S400) the presence of a user's hand and the posture of the hand
- dispensing (S410) a hand disinfectant medium comprising UV, IR, or visible light absorbing material onto the hand of the user in a specific dose,
- illuminating (S420) the hand by the UV, visible or IR light,
- recording (S430) one or more digital images of the hand by from at least two sides of the hands,
- based on the recorded images, generating (S440) intensity information for the hand, wherein said intensity information provides information on the extent of hand surface area covered by the disinfectant medium,
- comparing (S450) the generated intensity information with threshold intensity information which corresponds to an acceptable hand sanitation level, and
- based on a result of the comparison, determining (S460) whether or not the quality of the hand disinfection is acceptable.

11. The method of claim 10, further comprising the step of identifying (S405) the user by means of RFID, technology and wherein the step of dispensing (S410) a hand disinfectant medium comprises dispensing the medium in a user-specific dose.

12. The method of claim 10 or 11, further comprising the step of displaying the result of the hand disinfection quality control test for the user on a display and/or generating a respective audio signal for the user to indicate the success or failure of the hand disinfection quality control test.

13. The method of any one of claims 10 to 12, further comprising the step of forwarding the recorded images and/or the test results to an external device for further processing.

14. A computer program product containing instructions which, when executed by the apparatus according to any one of claims 1 to 9, cause the apparatus to perform the method according to any one of claims 10 to 13.

## Patentansprüche

1. Vorrichtung (10) zur Händedesinfektions-Qualitätskontrolle, die Vorrichtung umfassend
- ein Gehäuse (12), wobei innerhalb des Gehäuses (12) die Vorrichtung umfasst:
- einen Handprüfhohlraum (80), der auf einer Vorderseite des Gehäuses ausgebildet und zur Aufnahme einer Hand eines Benutzers ausgelegt ist,
- eine Vielzahl von Lichtquellen (14), die angeordnet sind, um Ultraviolett-, UV-, oder sichtbares Licht oder Infrarot-, IR-, Strahlung in einen Innenraum des Handprüfhohlraums zu leiten,
- mindestens eine Digitalkamera (16), die in dem Handprüfhohlraum angeordnet und konfiguriert ist, um Bilder im UV-, sichtbaren oder IR-Spektrum aufzunehmen,
- einen Handnäherungssensor (18), der konfiguriert ist, um das Vorhandensein einer Hand eines Benutzers innerhalb des Handprüfhohlraums zu erfassen,
- einen Behälter (22) zum Speichern eines Desinfektionsmediums,
- einen Spender (20) für eine dosierte Verabreichung des Desinfektionsmediums,
- eine Prozessoreinheit (30) und
- eine interne Datenspeichereinheit (24),
wobei die Vorrichtung auf der Außenfläche des Gehäuses (12) weiter umfasst:
- Eingabemittel (40) zum lokalen Steuern des Betriebs der Vorrichtung, und
- Anzeigemittel (50),
wobei das Desinfektionsmedium ein UV- oder IR-absorbierendes Material oder ein phosphoreszierendes Material oder ein chemilumineszierendes Material enthält, und
wobei die Prozessoreinheit (30) konfiguriert ist,
- um aus den von der mindestens einen Kamera (16) aufgezeichneten Bildern Intensitätsinformationen für die Hand zu erzeugen, wobei die Intensität Informationen über das Ausmaß der von dem Desinfektionsmedium bedeckten Handoberfläche bereitstellt,
- um die Intensitätsinformationen unter Verwendung eines vorbestimmten oder benutzerspezifischen Bewertungsalgorithmus zum Bestimmen des akzeptablen Händehygienisierungsgrades zu verarbeiten, und
- um basierend auf der Intensitätsinformationsverarbeitung zu bestimmen, ob die Qualität der Desinfektion der geprüften Hand akzeptabel ist oder nicht.

2. Vorrichtung nach Anspruch 1, wobei die Wände des Prüfhohlraums (80) aus einem UV-, sichtbaren Licht- oder IR-absorbierenden Material hergestellt oder mit einem UV-, sichtbaren Licht- oder IR-absorbierenden Material bedeckt sind oder wobei ihre UV-, sichtbaren Licht- oder IR-Reflexionseigenschaften deutlich von denen der Haut einer menschlichen Hand abweichen.

3. Vorrichtung nach Anspruch 1 oder 2, wobei die Eingabemittel (40) und die Anzeigemittel (50) als integrierter Berührungsbildschirm ausgebildet sind.

4. Vorrichtung nach einem der Ansprüche 1 bis 3, weiter umfassend einen oder mehrere Statusanzeigen, um visuelle oder akustische Statussignale zu erzeugen, die Betriebsarten der Vorrichtung und/oder Ergebnisse der Desinfektions-Qualitätskontrollprüfung darstellen.

5. Vorrichtung nach einem der Ansprüche 1 bis 4, weiter umfassend eine Schnittstelleneinheit (70) zum Verbinden der Vorrichtung mit einer externen Prozessoreinrichtung über eine drahtgebundene oder drahtlose Verbindung.

6. Vorrichtung nach einem der Ansprüche 1 bis 5, weiter umfassend ein Benutzeridentifikationsmodul zum Identifizieren des Benutzers, dessen Hand einer Desinfektions-Qualitätskontrolle unterzogen wird.

7. Vorrichtung nach Anspruch 6, wobei das Benutzeridentifikationsmodul auf der Personenidentifikationstechnologie basiert, die ausgewählt ist aus der Gruppe von: Magnetkarten-, Smartcard-, RFID-, NFC-, Fingerabdruck-, Ultraschall- oder Infrarot- (IR-) Identifikation.

8. Vorrichtung nach einem der Ansprüche 1 bis 7, wobei ein visuelles und akustisches Warnmittel mit der Vorrichtung verbunden ist, wobei das Warnmittel konfiguriert ist, um die umgebenden Personen zu benachrichtigen, wenn das Handhygieneleistung eines Benutzers die Anforderungen nicht erfüllt hat.

9. Vorrichtung nach einem der Ansprüche 3 bis 8, wobei sie weiter einen Berührungsbildschirm enthält, um Zugang zu ihrer internen Software für Service und Wartung zu ermöglichen.

10. Verfahren zur Durchführung einer Händedesinfektions-Qualitätskontrolle an einer Vorrichtung nach einem der Ansprüche 1 bis 9, wobei das Verfahren die Schritte umfasst von:
- Erfassen (S400) des Vorhandenseins der Hand eines Benutzers und der Haltung der Hand
- Abgeben (S410) eines Händedesinfektionsmediums, das ein UV-, IR- oder sichtbares Licht absorbierendes Material umfasst, auf die Hand des Benutzers in einer bestimmten Dosis,
- Beleuchten (S420) der Hand durch UV-, sichtbares oder IR-Licht,
- Aufzeichnen (S430) eines oder mehrerer digitaler Bilder der Hand von mindestens zwei Seiten der Hände,
- basierend auf den aufgezeichneten Bildern, Erzeugen (S440) von Intensitätsinformationen für die Hand, wobei die Intensitätsinformationen Informationen über das Ausmaß der Handoberfläche liefern, die durch das Desinfektionsmedium bedeckt ist,
- Vergleichen (S450) der erzeugten Intensitätsinformationen mit Schwellenwert-Intensitätsinformationen, die einem akzeptablen Hygienisierungsgrad der Hand entsprechen, und
- basierend auf einem Ergebnis des Vergleichs, Bestimmen (S460), ob die Qualität der Händedesinfektion akzeptabel ist oder nicht.

11. Verfahren nach Anspruch 10, weiter umfassend den Schritt des Identifizierens (S405) des Benutzers mittels RFID-Technologie, und wobei der Schritt des Abgebens (S410) eines Händedesinfektionsmediums das Abgeben des Mediums in einer benutzerspezifischen Dosis umfasst.

12. Verfahren nach Anspruch 10 oder 11, weiter umfassend den Schritt des Anzeigens des Ergebnisses des Händedesinfektions-Qualitätskontrollprüfung für den Benutzer auf einer Anzeige und/oder des Erzeugens eines entsprechenden Audiosignals für den Benutzer, um den Erfolg oder Misserfolg der Händedesinfektions-Qualitätskontrollprüfung anzuzeigen.

13. Verfahren nach einem der Ansprüche 10 bis 12, weiter umfassend den Schritt der Weiterleitung der aufgezeichneten Bilder und/oder der Testergebnisse an eine externe Einrichtung zur weiteren Verarbeitung.

14. Computerprogrammprodukt, das Anweisungen enthält, die, wenn sie von der Vorrichtung nach einem der Ansprüche 1 bis 9 ausgeführt werden, bewirken, dass die Vorrichtung das Verfahren nach einem der Ansprüche 10 bis 13 durchführt.

## Revendications

1. Appareil (10) pour le contrôle de qualité de désinfection des mains, l'appareil comprenant :
- un boîtier (12), dans lequel, au sein du boîtier (12), l'appareil comprend :
- une cavité d'inspection de mains (80) formée sur une face avant du boîtier et qui est à même de recevoir une main d'un utilisateur,
- une pluralité de sources de lumière (14) agencées pour diriger un rayonnement d'ultraviolet, UV, ou de lumière visible ou d'infrarouge, IR, vers un espace interne de ladite cavité,
- au moins un appareil photographique (16) agencé dans la cavité d'inspection de mains et configuré pour capturer des images dans le spectre UV, visible ou IR,
- un capteur de proximité des mains (18) configuré pour détecter la présence d'une main d'un utilisateur dans ladite cavité d'inspection de mains,
- un récipient (22) pour stocker un agent désinfectant,
- un distributeur (20) pour un dosage mesuré de l'agent désinfectant,
- une unité de processeur (30) et
- une unité de stockage de données internes (24),
dans lequel, sur la surface externe du boîtier (12), l'appareil comprend en outre :
- des moyens d'entrée (40) pour commander localement le fonctionnement de l'appareil et
- des moyens d'affichage (50),
dans lequel l'agent désinfectant contient un matériau absorbant les UV ou les IR ou un matériau phosphorescent ou un matériau chimioluminescent, et
dans lequel l'unité de processeur (30) est configurée :
- pour générer des informations d'intensité pour la main à partir des images enregistrées par le au moins un appareil photographique (16), ladite intensité fournissant des informations sur le degré de couverture de surface de la main par l'agent désinfectant,
- pour traiter lesdites informations d'intensité en utilisant un algorithme d'évaluation prédéterminé ou spécifique à l'utilisateur afin de déterminer le niveau acceptable d'hygiène des mains et,
- sur la base dudit traitement des informations d'intensité, déterminer si la qualité de désinfection de la main inspectée est ou non acceptable.

2. Appareil selon la revendication 1, dans lequel les parois de la cavité d'inspection (80) sont constituées d'un matériau absorbant les UV, la lumière visible ou les IR ou revêtues d'un matériau absorbant les UV, la lumière visible ou les IR, ou elles ont leurs propriétés de réflexion des UV, de la lumière visible ou des IR notablement différentes de celles de la peau d'une main humaine.

3. Appareil selon la revendication 1 ou 2, dans lequel lesdits moyens d'entrée (40) et lesdits moyens d'affichage (50) se présentent sous la forme d'un écran tactile intégré.

4. Appareil selon l'une quelconque des revendications 1 à 3, comprenant en outre un ou plusieurs indicateurs d'état pour produire des signaux d'état visuels ou audibles représentant des modes de fonctionnement de l'appareil et/ou des résultats d'essai de contrôle de qualité de désinfection.

5. Appareil selon l'une quelconque des revendications 1 à 4, comprenant en outre une unité d'interface (70) pour connecter l'appareil à un dispositif de processeur externe via une interconnexion câblée ou sans fil.

6. Appareil selon l'une quelconque des revendications 1 à 5, comprenant en outre un module d'identification d'utilisateur pour identifier l'utilisateur dont la main est soumise à un contrôle de qualité de désinfection.

7. Appareil selon la revendication 6, dans lequel le module d'identification d'utilisateur est basé sur la technologie d'identification personnelle choisie dans le groupe des identifications suivantes : par carte magnétique, par carte à mémoire, par RFID, par NFC, par empreintes digitales, par ultrasons ou par infrarouges (IR).

8. Appareil selon l'une quelconque des revendications 1 à 7, dans lequel un moyen d'alerte visuel et audible est connecté à l'appareil, ledit moyen d'alerte étant configuré pour notifier aux personnes environnantes si une hygiène des mains de l'utilisateur n'a pas réussi à répondre aux exigences.

9. Appareil selon l'une quelconque des revendications 3 à 8, dans lequel il contient en outre un écran tactile pour fournir un accès à son logiciel interne pour le service et l'entretien.

10. Procédé pour réaliser un contrôle de qualité de désinfection des mains sur un appareil selon l'une quelconque des revendications 1 à 9, le procédé comprenant les étapes consistant à :
- détecter (S400) la présence d'une main d'utilisateur et la posture de la main,
- distribuer (S410) un agent désinfectant des mains comprenant un matériau absorbant les UV, les IR ou la lumière visible sur la main de l'utilisateur dans une dose spécifique,
- éclairer (S420) la main par la lumière UV, visible ou IR,
- enregistrer (S430) une ou plusieurs images numériques de la main depuis au moins deux côtés des mains,
- sur la base des images enregistrées, générer (S440) des informations d'intensité pour la main, dans lequel lesdites informations d'intensité fournissent des informations sur le degré de couverture de la surface manuelle de la main par l'agent désinfectant,
- comparer (S450) les informations d'intensité générées à des informations d'intensité de seuil qui correspondent à un niveau d'hygiène manuel acceptable et,
- sur la base d'un résultat de la comparaison, déterminer (S460) si la qualité de la désinfection des mains est ou non acceptable.

11. Procédé selon la revendication 10, comprenant en outre l'étape d'identification (S405) de l'utilisateur au moyen de la technologie RFID et dans lequel l'étape de distribution (S410) d'un agent désinfectant des mains comprend la distribution de l'agent en dose spécifique à l'utilisateur.

12. Procédé selon la revendication 10 ou 11, comprenant en outre l'étape d'affichage du résultat de l'essai de contrôle de qualité de désinfection des mains pour l'utilisateur sur un appareil d'affichage et/ou de génération d'un signal audio respectif pour l'utilisateur afin d'indiquer le succès ou la défaillance de l'essai de contrôle de qualité de désinfection des mains.

13. Procédé selon l'une quelconque des revendications 10 à 12, comprenant en outre l'étape d'acheminement des images enregistrées et/ou des résultats d'essai à un dispositif externe pour un autre traitement.

14. Produit de programme d'ordinateur contenant des instructions qui, lorsqu'elles sont exécutées par l'appareil selon l'une quelconque des revendications 1 à 9, amènent l'appareil à effectuer le procédé selon l'une quelconque des revendications 10 à 13.
